# EUROPEAN PATENT SPECIFICATION

(11) **EP 3 150 934 B1**
(45) Date of publication and mention of the grant of the patent: **23.09.2020**
(21) Application number: 15799619.0
(22) Date of filing: 25.05.2015
(51) Int. Cl.: F24F 6/00, F24F 6/12, F24F 3/16, F24F 3/14, A61L 9/12

(54) **ULTRASONIC SPACE-STERILIZING HUMIDIFIER**
ULTRASCHALLRAUMSTERILISIERUNGSBEFEUCHTER
HUMIDIFICATEUR DE STÉRILISATION D'ESPACE À ULTRASONS

(30) Priority: 30.05.2014 JP 2014111847
(43) Date of publication of application: 05.04.2017
(73) Proprietor: Eco Factory Co., Ltd., Kumamoto 862-0950 (JP)
(72) Inventor: MURAKAMI Takanobu, Kumamoto-shi Kumamoto 862-0950 (JP)
(74) Representative: Isarpatent
(86) International application number: PCT/JP2015/064908
(87) International publication number: WO 2015/182548

(56) References cited:
- EP-A2- 1 832 817
- WO-A1-2006/095816
- JP-A- 2006 125 648
- JP-A- 2007 101 023
- JP-A- 2008 180 469
- JP-A- 2009 058 160
- JP-A- 2011 217 889
- JP-B1- 4 588 104
- JP-U- S5 952 317

## Description

### Technical Field

The present invention relates to an ultrasonic space-sterilizing humidifier that supplies humidified air containing hypochlorous acid, which exhibits an excellent sterilizing effect, to an interior space, and can humidify and sterilize large spaces such as patient rooms at medical facilities, etc.

### Background Art

A variety of health hazards have been identified in air environments, including influenza virus, pollen hypersensitivity allergy and PM2.5.

One countermeasure taken in poor air environments and especially in inhabited zones, is humidity control, which has a major effect on human health and is also highly effective for prevention of aerial infection by influenza virus, etc., and therefore household humidifiers are becoming commonplace in homes. Similarly, while the need for introducing humidifiers in welfare facilities or large-space facilities such as hospitals, day care centers, kindergartens and schools is also increasing, large-volume humidifiers that are able to withstand use in such facilities have not become common and instead, it is common to employ several household humidifiers in such facilities. However, with household humidifiers, the humidifying power is low and it is difficult to obtain the desired humidifying effect, while effort is also required for water supply and maintenance, and therefore demand is high for humidifiers that are specially designed for large spaces and that have high humidifying power and easy maintenance.

Incidentally, conventionally used humidifiers are largely classified according to their humidifying method, being classified into the 3 types of vaporizing types, steam types and ultrasonic types.

First, with vaporizing types, wind is blown onto a water-containing filter and moisture is vaporized at ordinary temperature, and because such types of humidifiers have simple structures and low power consumption, they are advantageous in terms of cost. However, these are associated with hygienic problems such as proliferation of saprophytic bacteria such as Legionella, or mold, on the filters or water tanks, while their humidifying power is also low, making them unsuitable for humidification of large spaces.

Steam types, on the other hand, employ a method of boiling water and releasing the generated steam into a fan, but such types of humidifiers exhibit excellent performance in terms of hygiene because sterilization is accomplished by boiling of saprophytic bacteria, etc., that are present in water. Nevertheless, due to cost and very high power consumption for boiling water, such types of humidifiers are also associated with serious problems in terms of operating cost, if they are to be used for humidification of large spaces.

Finally, ultrasonic types use an ultrasonic generator to create a mist from water to droplets with sizes of about 1 to 5 µm, and releasing them together with air using a fan, and such types of humidifiers are characterized by having high humidifying power and low power consumption. However, since an ultrasonic type operates at ordinary temperature similar to a vaporizing type, saprophytic bacteria, mold, etc., tend to proliferate in the water tank in which the humidifying water is stored, and such contaminants such as mold and saprophytic bacteria in the water tank ride on the mist and are released into the atmosphere, creating a problem in terms of hygiene.

When the above-described 3 types of humidifying methods are compared from the viewpoint of humidifying power and operating cost as humidifiers for large spaces, it maybe said that the ultrasonic type is the optimal humidifying method if the above-described problems of hygiene can be overcome, and in regard to this problem, a technique of adding hypochlorous acid to humidifying water is already being developed.

Hypochlorous acid is a chlorine-based disinfectant, and when contacted with bacteria, etc., it sterilizes by a powerful oxidizing effect, exhibiting a satisfactory disinfecting effect against a very wide range of different microorganisms and viruses. Therefore, if hypochlorous acid is added to humidifying water in an ultrasonic humidifier, it will be possible to limit proliferation of saprophytic bacteria, mold, etc., in the humidifying water. Furthermore, since hypochlorous acid has low persistence and is highly safe for the human body, methods of space sterilization have been proposed in which an aqueous hypochlorous acid solution is atomized directly into the air to inactivate microorganisms or viruses floating in the environmental space.

Thus, using hypochlorous acid water in the humidifying water of an ultrasonic humidifier can reduce proliferation of saprophytic bacteria, mold, etc., in the humidifying water, while also diffusing hypochlorous acid together with humidified air into the surrounding air, so that spaces can be humidified to obtain a space-sterilizing effect.

Patent Literature 1 is a publication describing an ultrasonic humidifier employing hypochlorous acid water, the ultrasonic humidifier using an electrolytic electrode in a water storage tank to generate hypochlorous acid. Also, Patent Literature 2 describes a technology in which an apparatus that releases an aqueous hypochlorous acid solution as a mist of fine particles by ultrasonic waves diffuses it through a diffusion tube to prevent the fine particles from becoming water droplets.

Patent Literature 3 describes an electrolyzed water mist generating unit mounted on an air cleaner and dissipates mist by clean air. The electrolyzed water mist generating unit comprises a water storage tank, at least a pair of electrode plates for electrolyzing water in the water storage tank to make electrolyzed water, a mist guiding pass that extends upwards from the storage tank, and a sirocco fan for blowing out the electrolyzed water mist.

Patent Literature 4 describes a humidifying apparatus comprising a steam humidifying unit or an ultrasonic humidifying unit, a humidifying air duct, a humidified air blowing port, and water collecting means, i.e., by liquefying steam or fog passing through a humidifying air path, the water flowing from the humidifying air duct to the humidified air blowing port is collected and discharged.

Patent Literature 5 describes an air purifier comprising a housing, an electrolyzed water generating device, an electrolytic water mist generating device, wherein the electrolyzed water mist generated by the mist generating device is discharged through a removable duct.

Patent Literature 6 describes an air filtering apparatus comprising an electrolytic water mist generator for generating electrolytic water mist from electrolytic water containing active oxygen species, and an air blowing unit for blowing air into the electrolytic water mist and blowing out air passed through the electrolytic water mist into a room.

As described above, adding hypochlorous acid to humidifying water in an ultrasonic humidifier is a highly effective means for solving the above-described hygienic problems associated with ultrasonic type humidifiers, and a space-sterilizing effect may also be anticipated, such that an ultrasonic type humidifier using hypochlorous acid can exhibit performance as a space-sterilizing humidifier.

### Citation List

### Patent Literature

Patent Literature 1: Japanese Published Unexamined Patent Application No. H10-281502
Patent Literature 2: Japanese Published Unexamined Patent Application No. 2000-300649
Patent Literature 3: JP 2009 058160 A
Patent Literature 4: JP 2008 180469 A
Patent Literature 5: JP 2011 217889 A
Patent Literature 6: EP 1 832 817 A2

### DISCLOSURE OF THE INVENTION

### Problems to be Solved by the Invention

The problem to be solved by the present invention is that of actively removing contaminants present in humidified air, which are a common problem when an ultrasonic space-sterilizing humidifier using an aqueous hypochlorous acid solution as described above has been designed with a large capacity to withstand application at facilities with large spaces, and of also maintaining a sufficient hypochlorous acid concentration in the humidifying water, to adequately exhibit not only a humidifying effect but also a space-sterilizing effect.

In this regard, the humidifier described in Patent Literature 2, for example, has a basic structure in which indoor air is drawn in and humidified air is discharged upward through an outer cylinder, and in particular it provides means that prevents aggregation of mist generated by an ultrasonic generator, but adsorption of water droplets can also take place inside the outer cylinder, and there being provided a structure for collecting the water droplet with a pan, the design is also such that the collected water is sent to the aqueous hypochlorous acid solution tank and reutilized.

However, because the ultrasonic humidifier is operated at ordinary temperature, a certain period of time is required to completely gasify the mist generated by the ultrasonic generator, and especially when it is attempted to generate a large amount of mist for large spaces, in the case of an ultrasonic humidifier having the construction described in Patent Literature 2, the very large amount of mist will aggregate, producing large-sized water droplets that become adsorbed in large amounts onto the inside wall of the air duct (outer cylinder), and it has been impossible to avoid dripping of the aqueous hypochlorous acid solution into the tank.

Moreover, as described above, while an aqueous hypochlorous acid solution can be used in the humidifying water to help limit proliferation of saprophytic bacteria in the humidifying water, there remains the separate issue of various types of contaminants harmful to human health, such as microscopic bacteria, mold spores, etc., that are also present in significant amounts in the air drawn in large amounts into the humidifier . Moreover, because the mist generated by an ultrasonic generator has a strong tendency to undergo accelerated aggregation around the contaminants as nuclei, forming water droplets, the contaminants drawn in from the surrounding air tend to become adsorbed on the inside wall of the air duct when incorporated into the water droplets. Thus, when the large amounts of water droplets containing such contaminants drip down through the inside wall of the air duct into the aqueous hypochlorous acid solution tank and are reutilized as humidifying water, the contaminants continue to accumulate in the tank. It is also possible that in the worst scenario, the contaminants become pulverized into fine particulates by the ultrasonic generator, potentially becoming released into the indoor room together with the humidified air.

Furthermore, since hypochlorous acid tends to actively react with contaminants and decompose, the contaminants that have accumulated in the tank notably reduce the concentration of hypochlorous acid in the tank. In addition, the water droplets themselves that drip into the tank also react with the contaminants, significantly lowering the efficacy of the hypochlorous acid, and therefore the hypochlorous acid concentration in the tank becomes diluted.

When an ultrasonic space-sterilizing humidifier using hypochlorous acid water is thus continuously used with increased capacity to withstand use in facilities having large spaces, it is impossible to avoid generation of large amounts of water droplets containing contaminants, and when the water droplets are reutilized as humidifying water, the contaminants contained in aspirated air accumulate in the humidifying water and produce the above-described adverse effects, while also lowering the concentration of hypochlorous acid in the humidified air, thus resulting in the problem that a space-sterilizing effect cannot be satisfactorily achieved.

Incidentally, among contaminants in air that has been drawn in as described above, the contaminants with large particle sizes, such as dirt, can be removed to some extent by using an intake filter, but in order to remove smaller contaminants such as viruses, it is necessary to use very expensive HEPA filters (High Efficiency Particulate Air Filters), and this undesirably increases the cost. Moreover, since higher performance filters are more prone to clogging, their use in large-capacity humidifiers with large air intake volumes has required frequent cleaning and replacement of the filters, and if such a filter is left clogged, the air throughput volume is reduced, the load on the air fan increases and the power consumption increases, potentially resulting in a problem that can result in breakdown, and therefore maintenance becomes an issue. Thus, large-capacity humidifiers with high intake volumes are poorly suitable for completely accomplishing removal of contaminants using filters.

It is an object of the present invention to solve the problems described above by providing a space-sterilizing humidifier with the optimal capacity for humidification and sterilization of large spaces such as patient rooms at medical facilities, as well as easy maintenance, making use of the nature of contaminants drawn in air to become easily adsorbed onto wall faces when incorporated into water droplets, to actively remove the contaminants in humidified air, and providing means for preventing water droplets that have reacted with contaminants and lost efficacy, from becoming included in the humidifying water, thereby allowing the hypochlorous acid concentration in the humidified air to be adequately maintained to exhibit a satisfactory space-sterilizing effect.

### Means for Solving the Problems

In order to achieve the object described above, the present invention is an ultrasonic space-sterilizing humidifier comprising a storage tank that stores an aqueous solution of hypochlorous acid, an aqueous hypochlorous acid solution production apparatus connected to the storage tank by tubing via a control valve, wherein the control valve is controlled by a controller to provide a constant supply to the storage tank depending on the amount of consumption of the aqueous solution of hypochlorous acid, an ultrasonic generator that is installed inside the storage tank and generates a hypochlorous acid-containing mist, an air duct that extends upwards from the storage tank, water-catching means provided near the bottom end of the air duct, which is a donut-shaped pan through the center section of which air passes, for collecting water droplets that have become adsorbed onto the inside wall of the air duct, blowing means for blowing the hypochlorous acid-containing mist into the air duct, and a discharge tube mounted on the water-catching means, by which water droplets that have become adsorbed onto the inside wall of the air duct are discharged to the outside so that they do not flow into the storage tank.

The second problem solving means is adsorption-promoting means that causes adsorption of water droplets on the surface, mounted in the air duct, at a location above the water-catching means.

The third problem solving means is the adsorption-promoting means as a longitudinal plate mounted on the inside of the air duct.

The fourth problem solving means is the adsorption-promoting means as a helical plate mounted on the inside of the air duct.

The fifth problem solving means is the drainage pipe being connected to a waste water tank.

Next, as explanation of the function of the first problem solving means, a fine mist containing hypochlorous acid generated by the ultrasonic generator rides the air from the blowing means and rises up through the air duct, and but if contaminants are floating in the air during that time, they will act as nuclei for aggregation of mist, forming water droplets. Heavy water droplets generated in this manner have a lower rising rate inside the air duct, and eventually become adsorbed onto the inside wall of the air duct. The water droplets including the contaminants subsequently fall through the inside wall of the air duct, being collected by the water-catching means near the bottom end of the air duct, and are discharged out of the air duct by the discharge tube. Since the contaminants in the moving air are thus removed, and the contaminants in the water droplets do not enter the storage tank and water droplets with reduced hypochlorous acid efficacy are not reutilized as humidifying water, there is no reduction in concentration of the aqueous solution of hypochlorous acid in the storage tank. Moreover, since the water-catching means is a donut-shaped pan through the center section of which air passes, air flow through the air duct is not impeded and water droplets including contaminants that have fallen through the inside wall of the air duct can be thoroughly collected and discharged.

With the second problem solving means, the adsorption-promoting means that adsorbs water droplets on the surface is mounted in the air duct, thereby allowing contaminant-including water droplets to be actively adsorbed in the adsorption-promoting means as well, in addition to the inside wall of the air duct. Furthermore, since the mounting location is a location higher than the water-catching means, water droplets adsorbed onto the adsorption-promoting means are reliably collected in the water-catching means and do not drip down into the storage tank.

With the third problem solving means, in which the water droplet adsorption-promoting means is a plate having a wide surface area, large amounts of water droplets incorporating contaminants are actively adsorbed on its surface and contaminants can be effectively removed from the humidified air, while the fact that the plate is longitudinal allows the humidified air to smoothly pass through without inhibiting airflow in the air duct.

With the fourth problem solving means, in which the water droplet adsorption-promoting means is a helical plate, it is possible to adsorb large amounts of water droplets on the wide surface, while a tornado-like helical vortex is generated in the humidified air in the air duct, such that the heavy water droplets that have aggregated around the contaminants as nuclei become separated outward by centrifugal force, allowing the contaminant-containing water droplets to be more effectively adsorbed.

Finally, with the fifth problem solving means, waste water containing contaminants is pooled in a separate waste water tank from the storage tank, allowing the waste water to be hygienically disposed of.

### Effects of the Invention

The space-sterilizing humidifier of the present invention as described above allows contaminants that are harmful to the human body to be effectively removed from humidified air even when an ultrasonic type humidifier using hypochlorous acid water has increased capacity. Moreover, since water-catching means is provided that prevents the removed water droplets incorporating contaminants from being included in the aqueous solution of hypochlorous acid in the storage tank, the storage tank can be constantly maintained in a clean state while there is also no dilution of the hypochlorous acid concentration by water droplets with reduced efficacy. It is thus possible to supply large amounts of clean, highly sterilized humidified air to living spaces, while high performance intake filters are not necessary and maintenance is facilitated, so that it becomes possible to realize large-capacity space-sterilizing humidifiers suitable for use in large spaces such as patient rooms of medical facilities, etc., at low cost.

### BRIEF DESCRIPTION OF THE DRAWINGS

Fig. 1 is a side view of an ultrasonic space-sterilizing humidifier representing an embodiment of the present invention.
Fig. 2 is a side view and a longitudinal section diagram of water-catching means representing an embodiment of the present invention.
Fig. 3 is a side view of exemplary water-catching means.
Fig. 4 is a side view and a longitudinal section diagram of water droplet adsorption-promoting means representing an embodiment of the present invention.
Fig. 5 is a side view and a longitudinal section diagram of water droplet adsorption-promoting means representing another embodiment of the present invention.
Fig. 6 is a side view representing an embodiment of the present invention, based on a duct system.

### Best Mode for Carrying Out the Invention

The present invention will now be described by way of embodiments thereof.

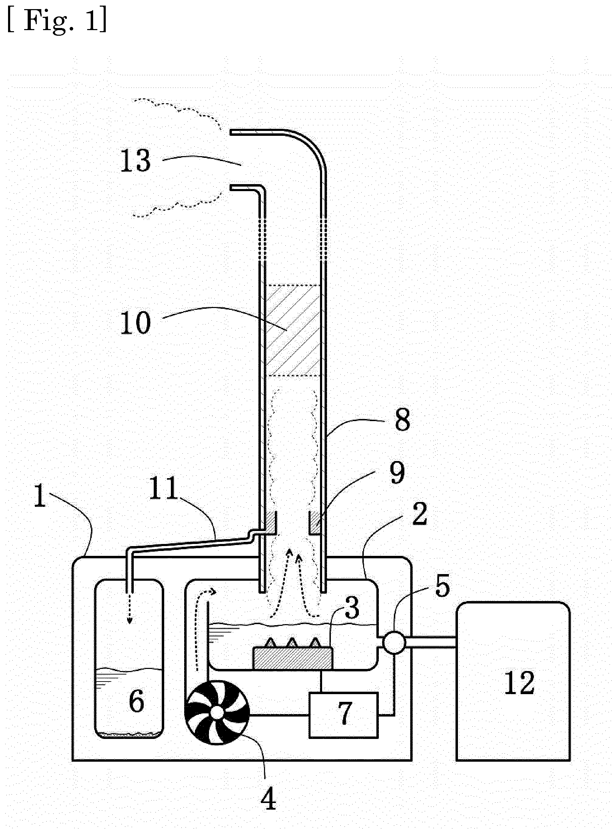

As shown in Fig. 1, the basic structure of the main body of the ultrasonic space-sterilizing humidifier of the present invention houses devices composing the humidifier main body, including a storage tank 2 that stores humidifying water comprising an aqueous solution of hypochlorous acid, an ultrasonic generator 3 installed in the storage tank 2, that generates hypochlorous acid-containing mist, blowing means 4 that blows the hypochlorous acid-containing mist, a control valve 5 that controls supply of the humidifying water, and a waste water tank 6 that stores waste water containing contaminants, in an enclosure 1, the blowing means 4, ultrasonic generator 3 and control valve 5 being integrally controlled by a controller 7. The present invention basically does not require an air filter, but for environments with high levels of dirt, a simple, easily maintained filter may be mounted on the gas inlet of the blowing means 4, for preliminary removal of contaminants with large particle sizes.

The air duct 8 connected to the upper wall of the storage tank 2 extends directly upward, the humidified air being blown in the transverse direction from a discharge port 13 at the top end. Also, near the bottom end of the air duct 8, there is mounted water-catching means 9 for catching water droplets that have become adsorbed onto the inside wall of the air duct 8, and a discharge tube 11 for discharge of water to the outside is mounted on the water-catching means 9, and is connected to a removable waste water tank 6. The enclosure 1 shown in Fig. 1 is directly installed at a suitable location of an indoor room, and since the humidifier of the present invention is designed to actively adsorb contaminant-containing water droplets in humidified air on the inside wall of the air duct 8, a longer air duct 8 produces a greater effect of removing contaminants. Therefore, when the enclosure 1 is directly installed in an indoor room, the height of the discharge port 13 is preferably designed to be located near the ceiling, ensuring an air duct 8 length of about 2 meters.

Since the humidifier of the present invention has an excellent sterilizing effect, it is preferably operated year-round for hygienic reasons, but certain periods, such as the summer season, will have excessive increase in humidity which may result in discomfort. Therefore, a known type of humidity sensor may be mounted to detect humidity, and the output controlled by a controller 7 to maintain a set desired humidity, allowing a hygienic, comfortable air environment to be maintained throughout the year.

As described in Patent Literature 1, the aqueous solution of hypochlorous acid used for the present invention may be obtained by direct production of hypochlorous acid in the storage tank 1 by known means, but for greater extendibility for adding additional humidifiers, and easy maintenance, an aqueous hypochlorous acid solution production apparatus 12 may be installed in a separate and independent manner from the humidifier main body housed in the enclosure 1, the aqueous solution of hypochlorous acid produced by the aqueous hypochlorous acid solution production apparatus 12 being supplied to the humidifier main body, as shown in Fig. 1. In the humidifier shown in Fig. 1, therefore, a design is employed in which the aqueous hypochlorous acid solution production apparatus 12 and the storage tank 2 are connected by tubing via a control valve 5, and the control valve 5 is controlled by a controller 7 to provide a constant supply to the storage tank 2 depending on the amount of consumption of the aqueous solution of hypochlorous acid. Also, the time required for water supply can be reduced if water is directly supplied to the aqueous hypochlorous acid solution production apparatus 12 through a water pipe.

Regarding hypochlorous acid, the bactericidal activity of hypochlorous acid is pH-dependent, its maximum bactericidal activity being exhibited in the weakly acidic range of pH 5 to 6, which is not harmful to the human body. Moreover, hypochlorous acid is an unstable substance and undergoes decomposition as time progresses, while it also has poor persistence as the presence of contaminants that produce organic materials or metal ions in aqueous solution accelerate its decomposition, and therefore it is a well-known microbicide that is widely used for sterilization of tap water and food, being a disinfectant with high safety for the human body.

Moreover, since the sterilizing property is by oxidation reaction alone, being a very simple action of removing electrons from substances (oxidizing them), its targets include a wide range of microorganisms, viruses, allergens and odorous substances, and it does not result in resistant microbes. For example, for food poisoning, it has inactivating potency against 0-157, *Salmonella* bacteria, *Legionella* bacteria, norovirus, influenza virus, multidrug-resistant microbe (MRSA), etc., and even allows sterilization of spore forming bacteria (such as *Bacillus cereus* and *Bacillus anthracis*), for which ethanol and invert soaps are ineffective. It also has inactivating potency against pollen hypersensitivity allergens, and its properties suggest that it is also capable of inactivating various other types of allergens. It also has very high ability to decompose odorous substances and can powerfully oxidize ammonia, methylmercaptan, etc., and is therefore an optimal chemical agent for use in space sterilization.

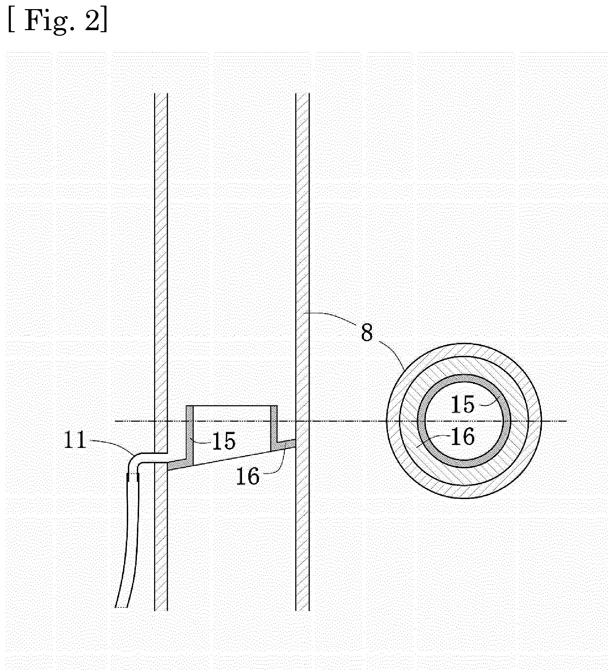

A mode of the water-catching means of the present invention will now be described. The water-catching means of the present invention is provided near the bottom end of the air duct 8 in order to collect water droplet containing contaminants, that have been adsorbed onto the inside wall of the air duct 8, and the water-catching means shown in Fig. 2 forms a donut-shaped pan, by an inner tube 15 with a smaller diameter than the inside wall of the air duct 8, and a base plate 16 blocking the bottom section of the gap formed between the inside wall of the air duct 8 and the inner tube 15, the interior of the inner tube 15 allowing free passage of gas.

When water droplets including contaminants become adsorbed on the inside wall of the air duct 8 above the pan, they fall down through the inside wall and are thus collected in the pan, and since the drainage pipe 11 is connected to the pan, the water droplets pooled in the pan can be discharged to the outside together with the contaminants, without flowing into the storage tank 2. If the base plate 16 is inclined to the side of connection with the drainage pipe 11 as shown in Fig. 2, the water can be smoothly directed to the drainage pipe 11.

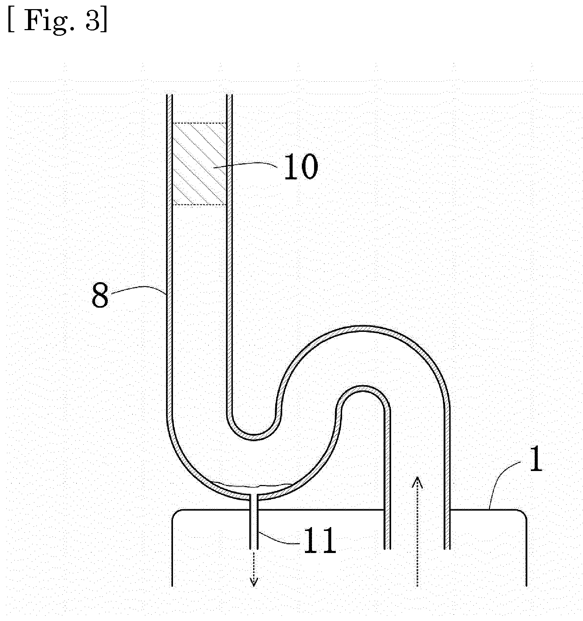

The water-catching means shown in Fig. 3 will now be described. For the water-catching means, two mutually inverted U-shaped tubes are combined near the bottom end of the air duct 8, forming an overall curved lateral S-shape, or "S-shaped trap," and the drainage pipe 11 is connected at the lowermost part of the U-shape at the lower part of the S-shaped trap.

In this case as well, similar to the pan described in Fig. 2, all of the water droplets including contaminants that have fallen through the inside wall of the air duct 8 collect at the location of the S-shaped trap that is connected to the drainage pipe 11, so that they can all be discharged to the outside without the contaminant-containing water droplets flowing into the storage tank 2.

When directly provided in the air duct 8 at the location where the water-catching means is provided, it is preferably located as near as possible to the bottom end of the air duct 8, and especially when providing a donut-shaped pan as shown in Fig. 2, it does not need to be provided directly in the air duct 8 but may be provided directly in the main body section of the humidifier, at a lower location than the bottom end of the air duct 8.

The drainage pipe 11 is directly connected to a sink pipe, etc., in order to allow constant drainage of waste water, but if the freely removable waste water tank 6 is housed in the enclosure 1 and the drainage pipe 11 is connected to the waste water tank 6, as shown in Fig. 1, it is possible to hygienically dispose of the waste water while also removing the contaminants precipitated in the waste water tank 6 for measurement of the level of pollution of the air environment, or for detailed analysis of the contaminants to help prevent spread of infectious disease. The waste water tank 6 is easy to maintain if it can ensure a volume that can be processed once every month or so.

A mode of the water droplet adsorption-promoting means of the present invention will now be described. While the present invention allows a considerable portion of contaminant-containing water droplets to be adsorbed onto the inside wall of the air duct 8, if a humidifier with an even greater capacity is desired, it may not be possible to adequately ensure the air duct 8 length, and mere adsorption onto the inside wall of the air duct 8 maybe insufficient. In such cases, therefore, in order to more reliably remove contaminants, it is effective to mount the adsorption-promoting means that adsorbs the water droplets at a location of the air duct 8 further above the water-catching means, for active adsorption of the water droplets.

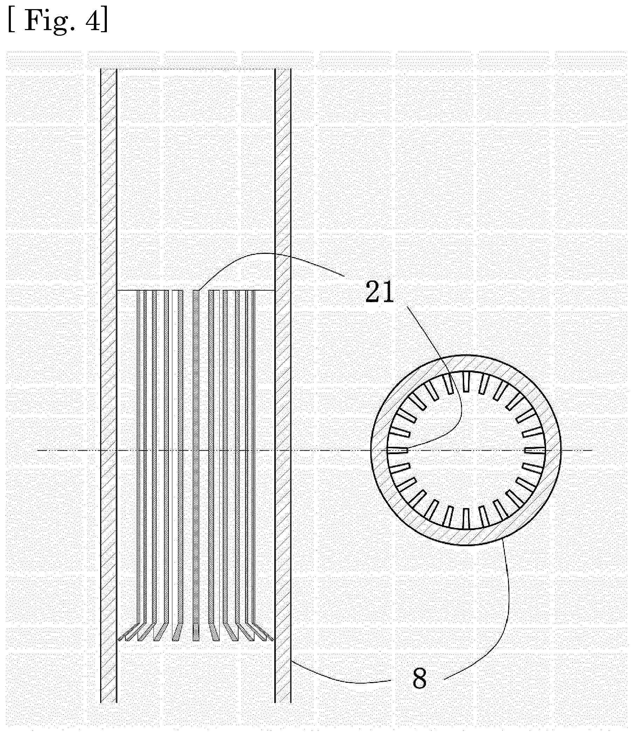

More specifically, adsorption of water droplets can be promoted by mounting adsorption-promoting means comprising a member that can adsorb the water droplets, such as a plate with a given surface area, at a location of the air duct 8 further above the water-catching means, to enlarge the section that adsorbs the water droplets. To first explain the adsorption-promoting means illustrated in Fig. 4, this adsorption-promoting means has a plurality of longitudinal plates 21 mounted so as to protrude in a concentric manner from the inside wall toward the center section of the air duct 8, to form a very wide adsorption area on the surfaces of the longitudinal plates 21, thereby allowing even more water droplets to be adsorbed. Also, since the bottom end of each longitudinal plate 21 is cut at an incline toward the inside wall side of the air duct 8, all of the water droplets that have been adsorbed on the longitudinal plate 21 are guided along the inside wall side of the air duct 8 and fall down through the inside wall of the air duct 8, and therefore even when the donut-shaped pan shown in Fig. 2 has been employed as the water-catching means, the water droplets passing through the center section do not drip down into the storage tank 2 and can be reliably collected by the water-catching means . Moreover, since the plates 21 are mounted in the longitudinal direction they do not inhibit airflow in the air duct 8.

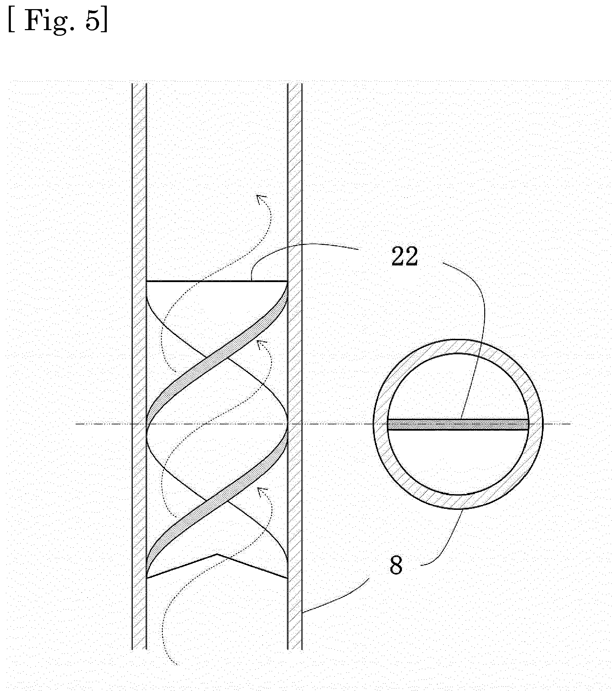

To explain the adsorption-promoting means illustrated in Fig. 5, this adsorption-promoting means has a helical plate 22, formed by twisting a rectangular plate, mounted inside the air duct 8, which likewise allows a wide adsorption area to be formed. Moreover, since the bottom end of the helical plate 22 is cut into an inverted V shape, all of the water droplets that have been adsorbed onto the helical plate 22 can be guided to the inside wall side of the air duct 8, similar to the mode illustrated in Fig. 4. When this adsorption-promoting means is mounted, a tornado-like helical vortex is generated in the airflow in the air duct 8 by the helical plate 22, such that heavy water droplets that have aggregated due to contaminants become separated outward by centrifugal force, allowing the water droplets to be efficiently adsorbed.

In all of the above-described adsorption-promoting means, the ability to remove contaminants is increased by the length of the section where the adsorption-promoting means is mounted, and therefore the plate length, etc., in the adsorption-promoting means may be appropriately adjusted for the capacity desired for the humidifier. The modes of the adsorption-promoting means are not limited to those illustrated in Fig. 4 and Fig. 5, as the effect of the adsorption-promoting means can be exhibited as long as it comprises a member allowing adsorption of water droplets on the surface.

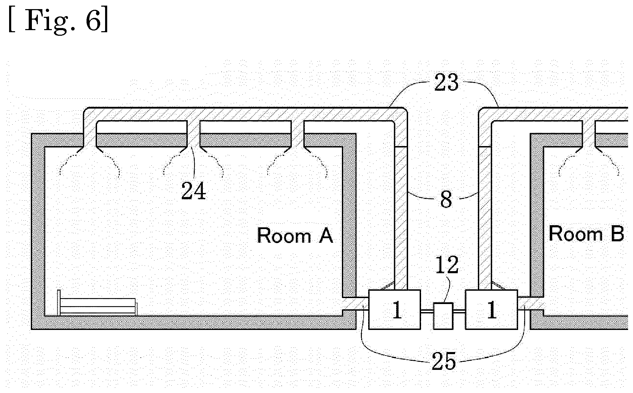

The mode illustrated in Fig. 1 assumes an enclosure 1 installed in an indoor room that performs direct atomization from the discharge port 13 into the indoor room for humidification and sterilization, but when it is installed in a large space, it may be difficult in some cases for the humidified air to reach to the corner areas of the space. In such cases, therefore, it is highly effective to employ a so-called duct system, as shown in Fig. 6, wherein the enclosure 1 of the humidifier is installed outside of the indoor room and air is sucked into the indoor room from an intake duct 25, while an air duct 8 is connected to a duct 23 to guide humidified air, which is discharged through a plurality of blowing ports 24 provided in the ceiling. The ultrasonic space-sterilizing humidifier of the present invention may also be applied to such a duct system, without any particular problems.

Due to its construction, a duct system is prone to adsorption of contaminants and is difficult to clean, and therefore has the disadvantage of being associated with proliferation of mold and saprophytic bacteria. In addition, if it is attempted to blow highly humid air through the duct, the humid air will further aid proliferation of mold and saprophytic bacteria, very possibly resulting in high levels of proliferation of mold and saprophytic bacteria in the duct. When humidified air is supplied with a duct system, therefore, it is necessary to blow highly clean humidified air with a powerful sterilizing property.

In this regard, since the humidifier of the present invention has contaminants such as mold and saprophytic bacteria removed in advance so that virtually no contaminants are included in the humidified air, the possibility of contaminants being adsorbed on the inside of the duct 23 is very low even when the air duct 8 is connected to the duct 23. Furthermore, even when mold or saprophytic bacteria are included in the duct 23 for some reason, the concentration of hypochlorous acid in the humidified air is sufficiently high so as to kill the mold or saprophytic bacteria, and thus even when the apparatus is idle for long periods there is extremely less potential for proliferation inside the duct 23, and the humidifier may be hygienically used.

When a duct system is employed, it is possible to effectively exhibit high extensibility by separately providing the aqueous hypochlorous acid solution production apparatus 12 from the humidifier main body housed in the enclosure 1. That is, as shown in Fig. 6, multiple humidifiers can be connected to a single aqueous hypochlorous acidsolution production apparatus 12 installed outside the indoor room, thereby allowing humidifiers for different rooms such as indoor room A and indoor room B to be easily and economically installed, for easier application to large-scale facilities.

### Industrial Applicability

As described above, since the ultrasonic space-sterilizing humidifier of the present invention allows humidified air containing no contaminants and containing hypochlorous acid, which has an excellent microbicidal effect, to be supplied in a large volume to an indoor space, for humidification and sterilization of the indoor space, and is also easy to maintain, it is especially suitable for use in large spaces with high requirements for humidification and sterilization, such as patient rooms at medical facilities.

### Reference Signs List

1: Enclosure, 2: storage tank, 3: ultrasonic generator, 4: blowing means, 5: control valve, 6: wastewater tank, 7: controller, 8: airduct, 9: water-catching means, 10: adsorption-promoting means, 11: drainage pipe, 12: aqueous hypochlorous acid solution production apparatus, 21: longitudinal plate, 22: helical plate, 23: duct.

## Claims

1. An ultrasonic space-sterilizing humidifier comprising:
a storage tank (2) that stores an aqueous solution of hypochlorous acid;
an aqueous hypochlorous acid solution production apparatus (12);
an ultrasonic generator (3) that is installed inside the storage tank (2) and generates a hypochlorous acid-containing mist;
an air duct (8) that extends upwards from the storage tank (2); water-catching means (9) provided near the bottom end of the air duct (8), which is a donut-shaped pan through the center section of which air passes, for collecting water droplets that have become adsorbed onto the inside wall of the air duct (8);
blowing means (4) for blowing the hypochlorous acid-containing mist into the air duct (8);
**characterized in that** the aqueous hypochlorous acid solution production apparatus (12) is connected to the storage tank (2) by tubing via a control valve (5), wherein the control valve (5) is controlled by a controller (7) to provide a constant supply to the storage tank (2) depending on the amount of consumption of the aqueous solution of hypochlorous acid; and **in that** it further comprises a discharge tube mounted on the water-catching means (9), by which water droplets that have become adsorbed onto the inside wall of the air duct (8) are discharged to the outside so that they do not flow into the storage tank (2).

2. The ultrasonic space-sterilizing humidifier according to Claim 1,
wherein adsorption-promoting means (10) that causes adsorption of water droplets on the surface is mounted in the air duct (8), at a location above the water-catching means (9).

3. The ultrasonic space-sterilizing humidifier according to Claim 2,
wherein the adsorption-promoting means (10) is a longitudinal plate (21) mounted inside the air duct (8).

4. The ultrasonic space-sterilizing humidifier according to Claim 2,
wherein the adsorption-promoting means (10) is a helical plate (22) mounted inside the air duct (8).

5. The ultrasonic space-sterilizing humidifier according to Claim 1,
comprising a waste water tank (6), the discharge tube being connected to the waste water tank (6).

## Patentansprüche

1. Ultraschallraumsterilisierungsbefeuchter, umfassend:
einen Speichertank (2), in dem eine wässrige Lösung von hypochloriger Säure bevorratet ist;
ein Gerät (12) zur Produktion einer wässrigen Lösung hypochloriger Säure;
einen Ultraschallerzeuger (3), der innerhalb des Speichertanks (2) angeordnet ist und einen Nebel erzeugt, der hypochlorige Säure enthält;
eine Luftleitung (8), die sich ausgehend von dem Speichertank (2) nach oben erstreckt;
wasserabfangende Mittel (9), die in der Nähe des unteren Endes der Luftleitung (8) bereitgestellt sind, wobei es sich um eine torusförmige Wanne handelt, durch deren Mitte Luft strömt, so dass Wassertröpfchen aufgefangen werden, die an der Innenwand der Luftleitung (8) adsorbiert wurden;
Blasmittel (4) zum Blasen des Nebels, der hypochlorige Säure enthält, in die Luftleitung (8);
**dadurch gekennzeichnet, dass** das Gerät (12) zur Produktion einer wässrigen Lösung von hypochloriger Säure mittels Schläuchen über ein Steuerungsventil (5) mit dem Speichertank (2) verbunden ist, wobei das Steuerungsventil (5) mittels eines Steuergeräts (7) derart gesteuert wird, dass in Abhängigkeit von der verbrauchten Menge der wässrigen Lösung von hypochloriger Säure eine konstante Versorgung des Speichertanks (2) erfolgt; und dadurch, dass es weiterhin einen Abführungsschlauch umfasst, der an den wasserabfangenden Mitteln (9) angebracht ist, wobei desselben Wassertröpfchen, die an der Innenwand der Luftleitung (8) adsorbiert wurden, derart nach außen abgeführt werden, dass sie nicht in den Speichertank (2) fließen.

2. Ultraschallraumsterilisierungsbefeuchter nach Anspruch 1, wobei ein adsorptionsförderndes Mittel (10), das eine Adsorption von Wassertröpfchen an der Oberfläche bewirkt, in der Luftleitung (8) angebracht ist, an einer Stelle oberhalb der wasserabfangenden Mittel (9).

3. Ultraschallraumsterilisierungsbefeuchter nach Anspruch 2, wobei es sich bei dem adsorptionsfördernden Mittel (10) um eine in Längsrichtung ausgebildete Platte (21) handelt, die innerhalb der Luftleitung (8) angebracht ist.

4. Ultraschallraumsterilisierungsbefeuchter nach Anspruch 2, wobei die es sich bei dem adsorptionsfördernden Mittel (10) um eine helixartig ausgebildete Platte (22) handelt, die innerhalb der Luftleitung (8) angebracht ist.

5. Ultraschallraumsterilisierungsbefeuchter nach Anspruch 1, wobei er einen Abwasserbehälter (6) umfasst, wobei der Abführungsschlauch an den Abwasserbehälter (6) angeschlossen ist.

## Revendications

1. Humidificateur de stérilisation d'espace à ultrasons, comprenant :
un réservoir de stockage (2) qui stocke une solution aqueuse d'acide hypochloreux ;
un appareil de production de solution aqueuse d'acide hypochloreux (12) ;
un générateur d'ultrasons (3) qui est installé à l'intérieur du réservoir de stockage (2) et qui génère un brouillard contenant de l'acide hypochloreux ;
un conduit d'air (8) qui s'étend vers le haut à partir du réservoir de stockage (2) ;
un moyen de captage d'eau (9) prévu près de l'extrémité inférieure du conduit d'air (8), qui est une cuvette en forme de beignet à travers la section centrale de laquelle passe de l'air, pour collecter les gouttelettes d'eau qui ont été adsorbées sur la paroi intérieure du conduit d'air (8) ;
un moyen de soufflage (4) pour souffler le brouillard contenant de l'acide hypochloreux dans le conduit d'air (8) ;
**caractérisé en ce que** l'appareil de production de solution aqueuse d'acide hypochloreux (12) est relié au réservoir de stockage (2) par un tube par l'intermédiaire d'une vanne de commande (5), dans lequel la vanne de commande (5) est commandée par un dispositif de commande (7) pour fournir une alimentation constante au réservoir de stockage (2) en fonction de la quantité de consommation de la solution aqueuse d'acide hypochloreux ; et **en ce qu'**il comprend en outre un tube d'évacuation monté sur le moyen de captage d'eau (9), par lequel les gouttelettes d'eau qui ont été adsorbées sur la paroi intérieure du conduit d'air (8) sont évacuées vers l'extérieur afin qu'elles ne s'écoulent pas dans le réservoir de stockage (2).

2. Humidificateur de stérilisation d'espace à ultrasons selon la revendication 1,
dans lequel un moyen favorisant l'adsorption (10) qui provoque l'adsorption de gouttelettes d'eau à la surface est monté dans le conduit d'air (8), à un emplacement situé au-dessus du moyen de captage d'eau (9).

3. Humidificateur de stérilisation d'espace à ultrasons selon la revendication 2,
dans lequel le moyen favorisant l'adsorption (10) est une plaque longitudinale (21) montée à l'intérieur du conduit d'air (8).

4. Humidificateur de stérilisation d'espace à ultrasons selon la revendication 2,
dans lequel le moyen favorisant l'adsorption (10) est une plaque hélicoïdale (22) montée à l'intérieur du conduit d'air (8).

5. Humidificateur de stérilisation d'espace à ultrasons selon la revendication 1,
comprenant un réservoir d'eaux usées (6), le tube d'évacuation étant relié au réservoir d'eaux usées (6).
